# EUROPÄISCHE PATENTSCHRIFT

(11) **EP 3 481 310 B1**
(45) Veröffentlichungstag und Bekanntmachung des Hinweises auf die Patenterteilung: **05.08.2020**
(21) Anmeldenummer: 17728063.3
(22) Anmeldetag: 08.05.2017
(51) Int. Cl.: A61B 17/16

(54) **MEDIZINISCHES INSTRUMENT ZUR ENTFERNUNG VON SPONGIOSA**
MEDICAL INSTRUMENT FOR REMOVAL OF SPONGY BONE
INSTRUMENT MÉDICAL DESTINÉ À L'ÉLIMINATION DE TISSUS SPONGIEUX

(30) Priorität: 06.07.2016 DE 102016212300
(43) Veröffentlichungstag der Anmeldung: 15.05.2019
(73) Patentinhaber: Eberle GmbH & Co. KG, 75449 Wurmberg (DE)
(72) Erfinder: EBERLE, Martin, 75449 Wurmberg (DE)
(74) Vertreter: Hoefer & Partner Patentanwälte mbB
(86) Internationale Anmeldenummer: PCT/EP2017/060882
(87) Internationale Veröffentlichungsnummer: WO 2018/007045

(56) Entgegenhaltungen:
- DE-B3-102014 214 404
- US-A1- 2002 165 612
- US-A1- 2015 018 886

## Beschreibung

Die vorliegende Erfindung betrifft ein medizinisches Instrument, insbesondere ein Ausräuminstrument, zur Entnahme von Spongiosa aus Knochen.

Knochen weisen neben der äußeren harten Knochenmasse in ihrem Inneren die sog. Spongiosa auf. Zur Behandlung verschiedenster Krankheiten kann es dabei notwendig sein, die Spongiosa im Inneren des Knochens auszuräumen. Hierbei offenbart beispielsweise die DE 201 09 331 U1 eine Vorrichtung zur Entnahme von Spongiosa, welche zylindrische Teilbereiche der Spongiosa aus dem Inneren des Knochens entnehmen kann. Hierdurch ist es jedoch nicht möglich, sämtliche Spongiosa aus dem Inneren des Knochens auszuräumen. Dies ist allerdings notwendig, wenn beispielsweise große Teile der Spongiosa nekrofiles Material aufweisen. Weiterhin offenbart die DE 102014214404 B3 ein Instrument zur Entnahme von Spongiosa gemäß der Präambel von Anspruch 1, welches ein deutlich vereinfachtes Ausräumen von nekrofiler Spongiosa mittels zweier sich gegenläufig öffnenden Schneidmessern ermöglichen. Allerdings hat sich herausgestallt, dass an Bereichen des Knochens, an welchen sich der Knochen erweitert, wie z.B. bei einem Hüftgelenksknochen, es schwierig sein kann, mit diesem medizinischen Instrument die befallene Spongiosa vollständig zu entfernen.

Es ist daher Aufgabe der vorliegenden Erfindung, ein medizinisches Instrument, insbesondere Ausräuminstrument, zur Entfernung von Spongiosa bereitzustellen, welches bei einfachem Aufbau und einfacher, kostengünstiger Herstellbarkeit ein sicheres und vollständiges Ausräumen der Spongiosa aus dem Inneren des Knochens ermöglicht.

Diese Aufgabe wird durch Instrument mit den Merkmalen des Anspruchs 1 gelöst. Die Unteransprüche zeigen bevorzugte Weiterbildungen der Erfindung.

Das erfindungsgemäße medizinische Instrument zur Entfernung von Spongiosa mit den Merkmalen des Anspruchs 1 weist den Vorteil auf, dass ein sicheres Ausräumen von Spongiosa aus dem Knocheninneren, insbesondere an Hinterschneidungen am Knochen, wie beispielsweise einem Hüftgelenkskopf, möglich ist. Hierbei kann das medizinische Instrument insbesondere durch eine relativ kleine Öffnung eingeführt werden und anschließend eine Schneideinrichtung ausgeschwenkt werden, welche die Spongiosa dann mit einem relativ großen Radius entfernen kann. Dabei ist das erfindungsgemäße medizinische Instrument sehr robust und stabil aufgebaut. Hierbei umfasst das medizinische Instrument eine Außenhülse und eine in der Außenhülse angeordnete Schubstange. Die Schubstange ist in der Außenhülse axial beweglich angeordnet. Ferner ist eine Schneideinrichtung an der Schubstange angeordnet und eine Kulissenführung vorgesehen, welche zwischen der Schubstange und der Schneideinrichtung angeordnet ist. Die Kulissenführung überführt dabei die Axialbewegung der Schubstange in eine Schwenkbewegung der Schneideinrichtung. Hierdurch wird die Schneideinrichtung vom medizinischen Instrument herausgeschwenkt und kann somit im Knochen ein größeres Volumen der Spongiosa entfernen. Zum Zurückziehen des medizinischen Instruments wird die Schubstange zurückgezogen und die Schwenkbewegung der Schneideinrichtung umgedreht, so dass die Schneideinrichtung wieder in die eingefahrene Ausgangsposition zurücküberführt wird und das medizinische Instrument problemlos durch eine kleine Einführöffnung aus dem Knochen herausgezogen werden kann. Weiterhin umfasst die Schneideinrichtung eine Kürette mit einem Grundkörper und einer ringförmigen Schneidenöse, an welcher eine Durchgangsöffnung vorgesehen ist. Die Schneidenöse ist somit ein ringförmiges, umlaufend geschlossenes Schneidelement, mit welchem insbesondere am distalen Ende Spongiosa abgetragen werden kann und durch die Durchgangsöffnung in der Schneidenöse abgeführt werden kann. Durch die Schwenkbarkeit der Schneidenöse können somit insbesondere auch Bereiche an Hinterschneidungen eines Knochens, wie z.B. bei einem sich erweiternden Hüftgelenkskopf, welcher im Wesentlichen kugelförmig ausgebildet ist, ermöglicht werden.

Vorzugsweise ist die Kürette als gekröpfte Kürette vorgesehen, wobei eine erste Längsachse der Schneidenöse abgewinkelt zu einer zweiten Längsachse des Grundkörpers angeordnet ist. Mit anderen Worten ist die Schneidenöse zum Grundkörper in einem Winkel angeordnet. Durch diese abgewinkelte Anordnung der Schneidenöse kann die Spongiosa insbesondere an Hinterschneidungsbereichen im Knochen besonders gut entfernt werden.

Vorzugsweise ist dabei zwischen der ersten und zweiten Längsachse ein stumpfer Winkel ausgeführt, vorzugsweise in einem Bereich von 165° bis 175° und der stumpfe Winkel beträgt besonders bevorzugt 170°.

Besonders bevorzugt weist die Schubstange an einem ersten Ende eine Schubstangenkopf auf, an welchem die Schwenkachse für die Schneideinrichtung angeordnet ist. Hierdurch ergibt sich ein besonders kompakter Aufbau.

Weiter bevorzugt weist die Kulissenführung eine in der Schneideinrichtung angeordnete Nut und einen an der Außenhülse angeordneten Bolzen auf, wobei der Bolzen in der Nut geführt ist. Hierdurch weist die Kulissenführung einen einfachen und betriebssicheren Aufbau auf.

Vorzugsweise ist die Nut bogenförmig ausgeführt, wobei die Nut besonders bevorzugt einen konstanten Radius aufweist.

Besonders gute Ausräumergebnisse werden dabei erreicht, wenn die Richtung der Kröpfung der Kürette in die gleiche Richtung gerichtet ist, in der die bogenförmige Nut verläuft.

Besonders bevorzugt ist eine Länge der Schneidenöse in Richtung der ersten Längsachse gleich groß wie eine Länge des Grundkörpers in Richtung der zweiten Achse. Diese Wahl der geometrischen Abmessungen der Längen des Grundkörpers und der Schneidenöse ermöglichen ein sehr kompaktes medizinisches Instrument.

Bevorzugt ist eine Verbindung zwischen der Schubeinrichtung und der Kürette mittels einer Stift-Bohrungs-Verbindung vorgesehen. Bevorzugt ist die Bohrung dabei in der Kürette vorgesehen und der Stift ist an der Schubeinrichtung vorgesehen. Alternativ ist der Stift an der Kürette ausgebildet und die Bohrung zur Aufnahme des Stifts ist an der Schubeinrichtung vorgesehen.

Gemäß einer weiteren bevorzugten Ausgestaltung der Erfindung sind an beiden Seiten der Durchgangsöffnung der Schneidenöse der Kürette Schneiden angeordnet. Dadurch kann das erfindungsgemäße Instrument in beiden Schwenkrichtungen Schneidvorgänge ausführen.

Die Schneideinrichtung ist bevorzugt an einem ersten Ende der Außenhülse angeordnet. Besonders bevorzugt ist dabei am ersten Ende der Außenhülse ein Schlitz vorgesehen, durch welchen hindurch die Schneideinrichtung aus der Außenhülse herausgeschwenkt wird.

Gemäß einer weiteren bevorzugten Ausgestaltung der Erfindung weist die Schubstange an einem zweiten Ende ein erstes Gewinde auf, welches mit einem zweiten Gewinde eines drehbaren Zylinders in Eingriff steht. Die beiden Gewinde bilden dabei eine Gewindeverbindung, um eine Drehbewegung des drehbaren Zylinders in die Axialbewegung der Schubstange umzusetzen. Der Zylinder weist dabei besonders bevorzugt eine Rändelung auf, so dass er mittels zweier Finger bedient werden kann.

Weiter bevorzugt weist die Gewindeverbindung eine vorbestimmte Selbsthemmung auf. Dadurch wird verhindert, dass während der Verwendung des medizinischen Instruments sich durch den Widerstand der Spongiosa die Schubstange dreht und das erste Gewinde der Schubstange wieder in das zweite Gewinde des drehbaren Zylinders zurückläuft, was zu einem Zurückschwenken der Schneideinrichtung führen würde.

Weiter bevorzugt ist ein Handgriff vorgesehen, wobei die Außenhülse besonders bevorzugt mittels einer Verriegelungsvorrichtung am Griff fixiert ist. Die Verriegelungsvorrichtung ist dabei eingerichtet, den Griff an der Außenhülse zu verriegeln und entriegeln. Hierdurch wird eine besonders einfache Handhabung des medizinischen Instruments ermöglicht. Besonders bevorzugt umfasst die Verriegelungsvorrichtung eine an der Entnahmehülse angeordnete Profilbuchse, eine am Griff vorgesehene Profilausnehmung und ein Schieberelement. Das Schieberelement kann in zwei Stellungen gebracht werden, wobei in der ersten Stellung die Außenhülse und der Griff miteinander verriegelt sind und in der zweiten Stellung die beiden entriegelt sind.

Nachfolgend wird ein bevorzugtes Ausführungsbeispiel der Erfindung unter Bezugnahme auf die begleitende Zeichnung im Detail beschrieben. In der Zeichnung ist:
- Fig. 1: eine schematische Seitenansicht eines medizinischen Instruments gemäß der Erfindung,
- Fig. 2: eine Schnittansicht von Fig. 1,
- Fig. 3: eine Teil-Seitenansicht des Instruments in nicht ausgeschwenktem Zustand,
- Fig. 4: eine Seitenansicht des Instruments in teilweise ausgeschwenktem Zustand,
- Fig. 5-8: Seitenansichten der Kürette,
- Fig. 9: eine Teil-Draufsicht der Kürette im montierten Zustand im medizinischen Instrument,
- Fig. 10: eine Ansicht des distalen Endes des medizinischen Instruments ohne Kürette, und
- Fig. 11: eine schematische Darstellung des medizinischen Instruments in einem aufgeschnittenen Hüftgelenkskopf.

Nachfolgend wird unter Bezugnahme auf die Figuren ein medizinisches Instrument 1 gemäß einem bevorzugten Ausführungsbeispiel der Erfindung im Detail beschrieben. Das medizinische Instrument 1 ist eingerichtet, um Spongiosa aus dem Knocheninneren zu entfernen bzw. einen Hohlraum im Spongiosagewebe zu erzeugen. Insbesondere soll mit dem medizinischen Instrument ein Ausschaben der Spongiosa aus dem Knocheninneren einfach und schnell ermöglicht werden.

Erfindungsgemäß umfasst das medizinische Instrument 1 eine Außenhülse 2 und eine Schubeinrichtung mit einer Schubstange 3, einer Verlängerungshülse 30, einem Gewindestift 31 und einem drehbaren Zylinder 7. Der Zylinder 7 weist dabei eine Rändelung an seinem Außenumfang auf, um zwischen zwei Fingern gedreht zu werden.

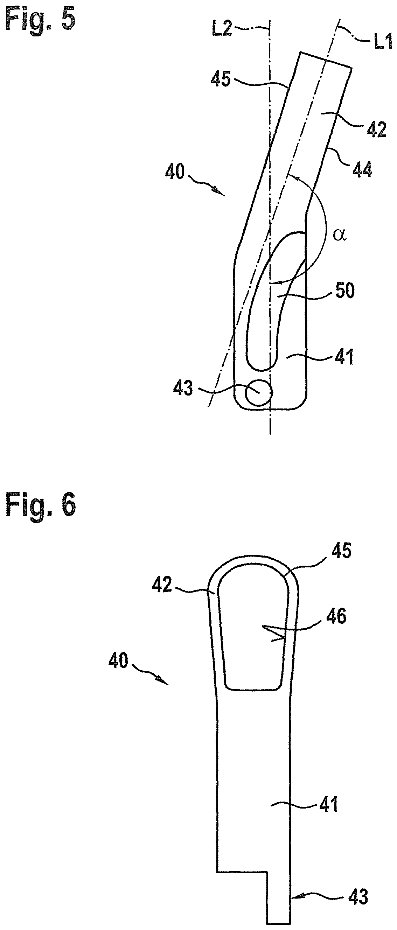

Die Schubstange 3 umfasst dabei einen Schubstangenkopf 32, an welchem eine Schwenkachse in Form eines Stifts 6 angeordnet ist. Weiterhin umfasst das medizinische Instrument 1 eine Schneideinrichtung 4 mit einer Kürette 40. Die Kürette 40 umfasst einen massiven Grundkörper 41 und eine Schneidenöse 42. Die Kürette 40 ist im Detail aus den Fig. 5 bis 8 ersichtlich.

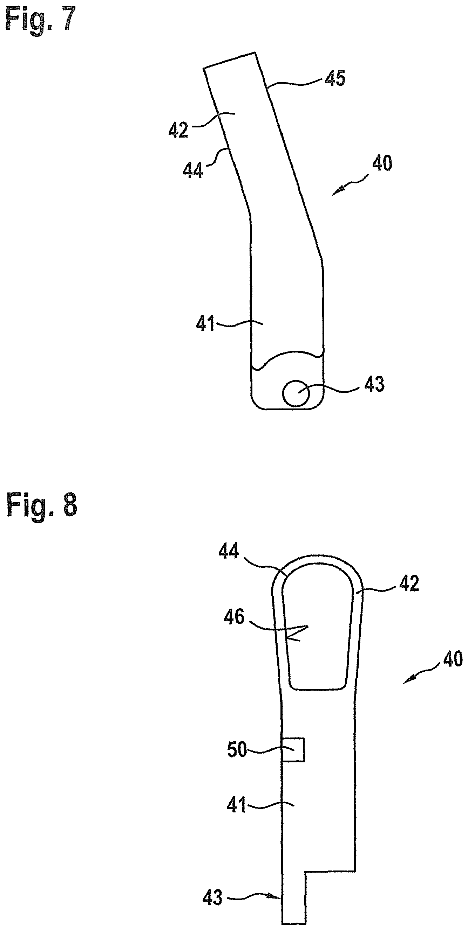

Wie insbesondere in den Fig. 6 und 8 dargestellt, ist die Schneidenöse 42 ein umlaufend geschlossenes Element mit einer ersten Schneidenkante 44 und einer zweiten Schneidenkante 45. Weiterhin ist an der Kürette am Grundkörper 41 eine Öffnung 43 zur Aufnahme des Stifts 6 vorgesehen.

Die Kürette 40 ist drehbar an der Schwenkachse 6 angeordnet.

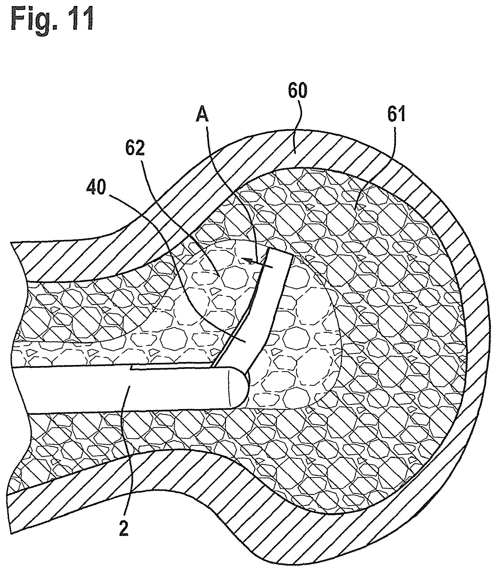

Die Kürette 40 ist als gekröpfte Kürette ausgebildet, wobei eine erste Längsachse L1 der Schneidenöse in einem Winkel α zu einer zweiten Längsachse L2 des Grundkörpers 41 abgewinkelt ist. Der Winkel α beträgt dabei ungefähr 170°. Durch diese gekröpfte Ausführung der Kürette 40 kann insbesondere, wie aus Fig. 11 ersichtlich ist, auch Spongiosa 61 an Hinterschneidungsbereichen 62 eines Knochens 60, wie beispielsweise einem Kugelkopf eines Hüftgelenks oder dgl., sicher entfernt werden.

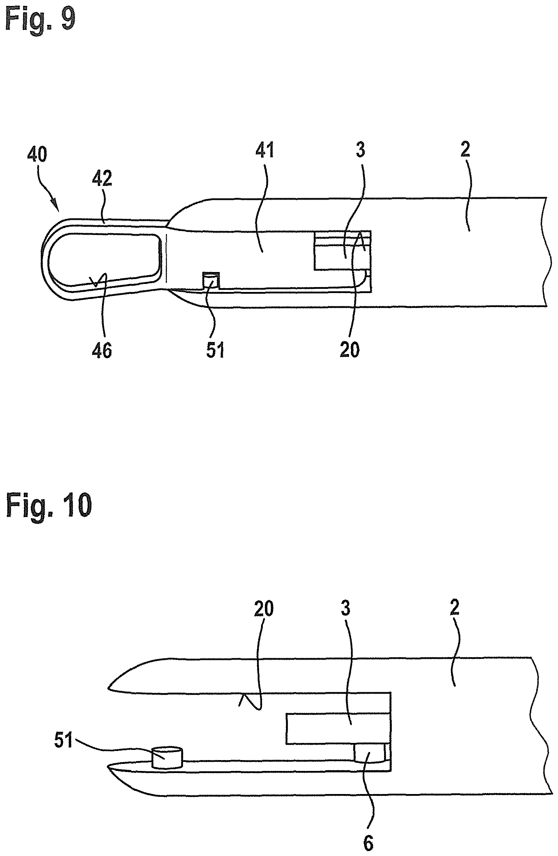

Weiterhin ist eine Kulissenführung 5 vorgesehen, welche eine Nut 50 und einen Bolzen 51 umfasst. Die Nut 50 ist im Grundkörper 41 der Kürette 40 gebildet und ist bogenförmig mit einem Radius R14 ausgeführt. Der Radius ist entlang der Nut 50 konstant. Wie aus Fig. 5 ersichtlich ist, beginnt die Nut 50 benachbart zur Öffnung 43 zur Aufnahme der Schwenkachse. Der Bolzen 51 ist an der Außenhülse 2 vorgesehen, und, wie aus Fig. 9 ersichtlich ist, welche den zurückgezogenen Zustand des medizinischen Instruments zeigt, am Ende der Nut 50 positioniert, welche bis zum Rand der Kürette 40 reicht. Somit ist der Bolzen 51 in zurückgezogenem Zustand sichtbar (vgl. Fig. 9). Dadurch kann ein Nutzer des medizinischen Instruments vor einem Einschieben des medizinischen Instruments in den Knochen sicher erkennen, dass sich das medizinische Instrument in der richtigen Einschiebeposition, d.h., der zurückgezogenen Position der Kürette, befindet.

Die Kröpfung der Kürette 40 ist somit derart ausgebildet, dass die Schneidenöse 42 in die gleiche Richtung gekröpft ist, in welcher die bogenförmige Nut verläuft (vgl. Fig. 5). Dadurch wird bei einer Axialbewegung in Axialrichtung X-X der Schubstange 3 eine Schwenkbewegung der Schneideinrichtung 4 bewirkt.

Wie aus den Fig. 6 und 8 ersichtlich ist, weist die Schneidenöse 42 eine Durchgangsöffnung 46 auf, so dass Spongiosa, welche durch die Schneidenöse 42 abgetrennt wurde, durch die Durchgangsöffnung hindurchgeführt werden kann. Somit kann gelöste Spongiosa sicher von dem Punkt, an welchem gerade der Schneidevorgang vorgenommen wird, abtransportiert werden.

Zwischen dem drehbaren Zylinder 7 und dem Gewindestift 31 ist eine Gewindeverbindung 8 mit einem ersten Gewinde und einem zweite Gewinde ausgebildet. Das erste Gewinde ist dabei am Gewindestift 31 angeordnet und das zweite Gewinde ist am Zylinder 7 angeordnet. Die Gewindeverbindung 8 bewirkt, dass bei einer Drehung des Zylinders 7 die Drehbewegung in eine Axialbewegung der Verlängerungshülse 30 und der Schubstange 3 in Axialrichtung X-X des medizinischen Instruments umgesetzt wird. Die beiden Gewinde sind dabei derart gewählt, dass eine vorgestimmte Selbsthemmung zwischen den beiden Gewinden vorhanden ist. Dadurch kann ein gewünschter, vorbestimmter ausgefahrener Zustand der Schneideinrichtung auch bei relativ großem Druck durch Drehen des Instruments am Handgriff während des Entfernens von Spongiosa beibehalten werden.

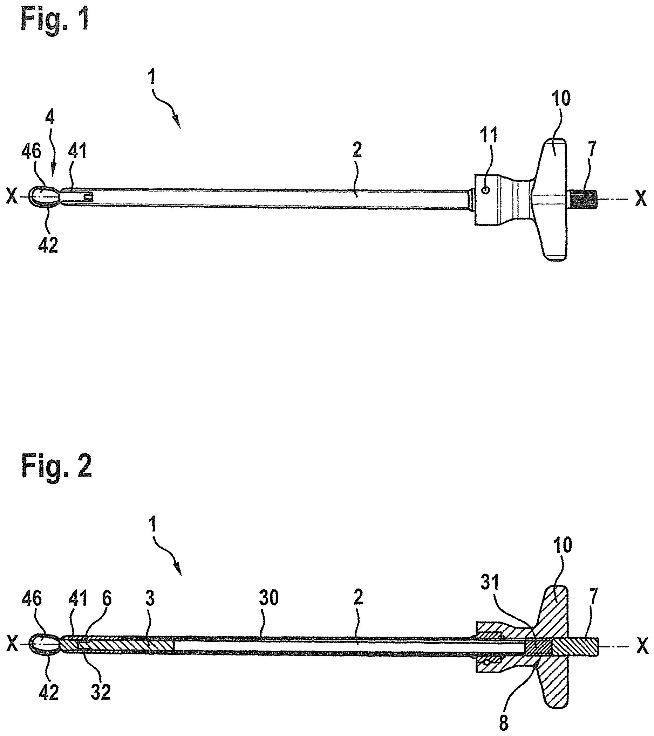

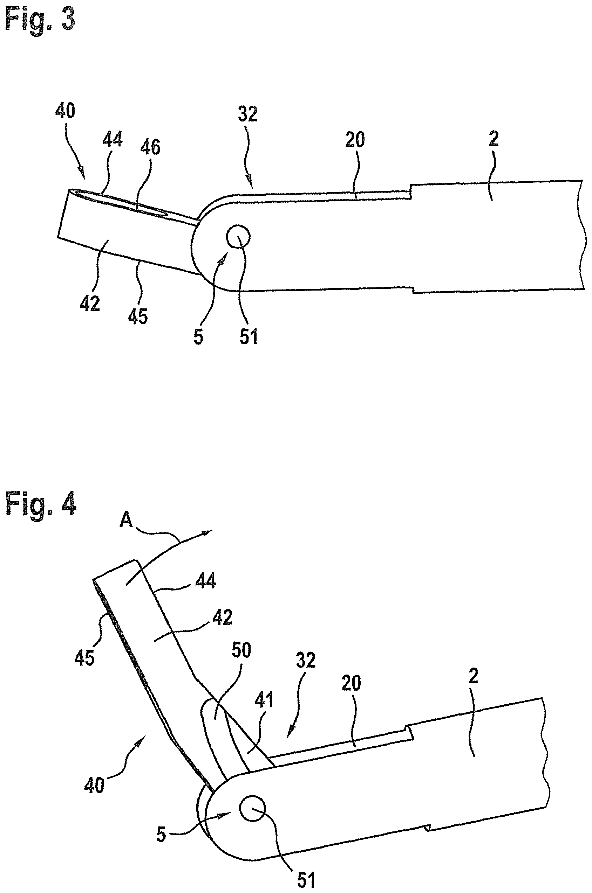

Die Kulissenführung 5 überführt die Axialbewegung der Schubstange 3 in eine Schwenkbewegung der Kürette 40. Die Fig. 1 bis 3 und 9 zeigen dabei den vollständig eingefahrenen Zustand der Kürette und die Fig. 4 und 11 einen ausgefahrenen Zustand der Kürette.

Zur besseren Handhabung des medizinischen Instruments ist ferner ein Griff 10 vorgesehen. Der Griff 10 weist eine wellenförmige Profilausnehmung auf, welche mit einer an der Außenhülse 2 angeordneten, entsprechend gebildeten wellenförmigen Profilbuchse im Eingriff steht. Mittels eines Schieberelements 11 kann der Griff 10 an der Außenhülse 2 verriegelt bzw. entriegelt werden.

Zur teilweisen Aufnahme der Kürette 40 weist die Außenhülse 2 an ihrem distalen Ende einen Schlitz 20 auf. Das distale Ende der Außenhülse 2 ist dabei entgegengesetzt zum Griff 10. Im Schlitz 20 ist dabei im zurückgezogenen Zustand, wie aus Fig. 3 ersichtlich, der Grundkörper 41 vollständig im Schlitz angeordnet.

Wie weiter aus Fig. 2 ersichtlich ist, ist die Schubstange 3 an einem Innenumfang der Außenhülse 2 geführt, so dass die Außenhülse 2 auch eine Führungsfunktion für die Schubeinrichtung bereitstellt.

Somit ist zwischen der Schubstange 3 und der Kürette 40 eine Stift-Bohrungs-Verbindung vorgesehen. Es sei angemerkt, dass selbstverständlich die Bohrung 43 statt in der Kürette auch in der Schubstange vorgesehen werden kann und der Stift 6 dann in der Kürette 40 vorgesehen werden kann.

Wie weiter aus den Fig. 5 und 7 ersichtlich ist, ist ungefähr eine erste Länge der Schneidenöse 42 in Richtung der ersten Längsachse L1 gleich lang wie eine zweite Länge des Grundkörpers in Richtung der zweiten Längsachse L2. Dadurch kann ein sehr kompakter und stabiler Aufbau der Kürette 40 sichergestellt werden.

Die Funktion des erfindungsgemäßen medizinischen Instruments 1 ist dabei wie folgt. Ausgehend vom eingefahrenen Zustand, welcher in den Fig. 1 und 3 dargestellt ist, wird an dem drehbaren Zylinder 7 gedreht. Dadurch wird die Drehbewegung des Zylinders 7 in eine Axialbewegung in Axialrichtung X-X der Schubeinrichtung umgesetzt. Da die Kürette 40 am Bolzen 51 in der Nut 50 geführt ist und der Bolzen 51 ortsfest an der Außenhülse 2 fixiert ist, wird durch die bogenförmige Nut 50 die Axialbewegung der Schubstange 3 in eine Schwenkbewegung der Kürette 40 umgesetzt. Dies ist in Fig. 4 durch den Pfeil A angedeutet.

Die Kulissenführung 5 ist somit zwischen der Schubeinrichtung, genauer der Schubstange 3 und der Kürette 40 angeordnet. Durch die Wahl des Radius des Bogens der Nut 50 kann dabei die Schwenkgeschwindigkeit in Abhängigkeit der Axialbewegung bestimmt werden. Um besonders feinfühlig agieren zu können, sollte ein möglichst geringer Schwenkwinkel pro vorbestimmter zurückgelegter axialer Länge vorgesehen werden.

Das erfindungsgemäße medizinische Instrument 1 weist, wie aus den Figuren ersichtlich ist, einen einfachen und kostengünstigen Aufbau auf. Dabei kann das medizinische Instrument sehr kompakt in den Knochen 60, nämlich in der vollständig eingefahrenen Position in den Knochen eingeführt werden und anschließend durch Drehen am Zylinder 7 herausgeschwenkt werden. Dadurch gelingt ein Ausräumen von Spongiosa 61 aus einem Knochen 60 in kürzerer Zeit und sehr effizienter Weise, da nicht nur zylindrische Stäbe aus der Spongiosa 61 entnommen werden können, wie in Fig. 11 schematisch gezeigt.

### Bezugszeichenliste

- 1: medizinisches Instrument
- 2: Außenhülse
- 3: Schubstange
- 4: Schneideinrichtung
- 5: Kulissenführung
- 6: Schwenkachse/Stift
- 7: drehbarer Zylinder
- 8: Gewindeverbindung
- 10: Griff
- 11: Schieberelement
- 20: Schlitz
- 30: Verlängerungshülse
- 31: Gewindestift
- 32: Schubstangenkopf
- 40: Kürette
- 41: Grundkörper
- 42: Schneidenöse
- 43: Öffnung für Schwenkachse
- 44: erste Schneidenkante
- 45: zweite Schneidenkante
- 46: Durchgangsöffnung
- 50: Nut
- 51: Bolzen
- 60: Knochen
- 61: Spongiosa
- 62: Hinterschneidungsbereich
- A: Ausschwenkbewegung
- L1: erste Längsachse
- L2: zweite Längsachse
- X-X: Axialrichtung des Instruments

## Patentansprüche

1. Medizinisches Instrument zur Entfernung von Spongiosa, umfassend:
- eine Außenhülse (2),
- eine in der Außenhülse (2) angeordnete Schubeinrichtung,
- wobei die Schubeinrichtung in Axialrichtung (X-X) des medizinischen Instruments beweglich in der Außenhülse (2) angeordnet ist,
- eine Schneideinrichtung (4), welche an der Schubeinrichtung angeordnet ist, und
- eine Kulissenführung (5), welche zwischen der Schubeinrichtung und der Schneideinrichtung (4) angeordnet ist, um die Axialbewegung der Schubeinrichtung in eine Schwenkbewegung der Schneideinrichtung (4) zu überführen,
- wobei die Schneideinrichtung (4) eine Kürette (40) mit einem Grundkörper (41) umfasst,
**dadurch gekennzeichnet, dass** die Kürette weiterhin eine ringförmige Schneidenöse (42) mit einer Durchgangsöffnung (46) umfasst.

2. Instrument nach Anspruch 1, **dadurch gekennzeichnet, dass** die Kürette (40) eine gekröpfte Kürette ist, wobei eine erste Längsachse (L1) der Schneidenöse (42) abgewinkelt zu einer zweiten Längsachse (L2) des Grundkörpers (41) angeordnet ist.

3. Instrument nach Anspruch 2, wobei zwischen der ersten Längsachse (L1) und der zweiten Längsachse (L2) ein stumpfer Winkel (a) vorgesehen ist, insbesondere in einem Winkelbereich von 165° bis 175°.

4. Instrument nach einem der vorhergehenden Ansprüche, **dadurch gekennzeichnet, dass** die Schubeinrichtung eine Schubstange (3) mit einem Schubstangenkopf (32) aufweist, an welchem die Schwenkachse (6) angeordnet ist.

5. Instrument nach einem der vorhergehenden Ansprüche, **dadurch gekennzeichnet, dass** die Kulissenführung (5) eine im Grundkörper (41) der Schneideinrichtung (4) angeordnete Nut (50) und einen an der Außenhülse (2) angeordneten Bolzen (51), welcher in der Nut geführt ist, umfasst.

6. Instrument nach Anspruch 5, wobei die Nut (50) eine bogenförmige Nut mit konstantem Radius ist.

7. Instrument nach Anspruch 6, wobei eine Kröpfung der Kürette (40) in die gleiche Richtung gerichtet ist, in welcher die bogenförmige Nut (50) verläuft.

8. Instrument nach einem der vorhergehenden Ansprüche, wobei eine Länge der Schneidenöse (42) in Richtung der ersten Längsachse (L1) ungefähr gleich groß ist wie eine Länge des Grundkörpers (41) in Richtung der zweiten Längsachse (L2).

9. Instrument nach einem der vorhergehenden Ansprüche, wobei eine Verbindung zwischen der Schubeinrichtung und der Kürette (40) eine Stift-Bohrungs-Verbindung umfasst.

10. Instrument nach einem der vorhergehenden Ansprüche, wobei an beiden Seiten der Durchgangsöffnung (46) der Kürette (40) Schneidenkanten (44, 45) vorgesehen sind.

## Claims

1. A medical instrument for removal of spongy bone, comprising:
an outer sleeve (2),
a pushing device (2) arranged in the outer sleeve,
wherein the pushing device is arranged in the outer sleeve (2) to be movable in the axial direction (X-X) of the medical instrument,
a cutting device (4) arranged on the pushing device, and
a motion link guide (5) arranged between the pushing device and the cutting device (4) for converting the axial movement of the pushing device into a pivoting movement of the cutting device (4),
wherein the cutting device (4) comprises a curette (40) with a base body (41),
**characterized in that** the curette further comprises an annular cutting eyelet (42) having a through opening (46).

2. The instrument according to claim 1, **characterized in that** the curette (40) is a cranked curette, wherein a first longitudinal axis (L1) of the cutting eyelet (42) is angled with respect to a second longitudinal axis (L2) of the base body (41).

3. The instrument according to claim 2, wherein an obtuse angle (α) is provided between the first longitudinal axis (L1) and the second longitudinal axis (L2), in particular in an angular range of 165° to 175°.

4. The instrument according to one of the preceding claims, **characterized in that** the pushing device comprises a push rod (3) with a push rod head (32) where the pivot axis (6) is arranged.

5. The instrument according to one of the preceding claims, **characterized in that** the motion link guide (5) comprises a groove (50) arranged in the base body (41) of the cutting device (4) and a stud (51) arranged on the outer sleeve (2), which is guided in the groove.

6. The instrument according to claim 5, wherein the groove (50) is an arcuate groove with a constant radius.

7. The instrument according to claim 6, wherein a crank of the curette (40) is directed in the same direction as the arcuate groove (50).

8. The instrument according to one of the preceding claims, wherein a length of the cutting eye (42) in the direction of the first longitudinal axis (L1) is approximately equal to a length of the base body (41) in the direction of the second longitudinal axis (L2).

9. The instrument according to one of the preceding claims, wherein a connection between the pushing device and the curette (40) comprises a pin-bore connection.

10. The instrument according to one of the preceding claims, wherein cutting edges (44, 45) are provided on both sides of the through opening (46) of the curette (40).

## Revendications

1. Instrument médical pour l'élimination de tissus spongieux, comprenant :
- une douille extérieure (2),
- un dispositif de poussée agencé dans la douille extérieure (2),
- dans lequel le dispositif de poussée est agencé mobile dans la douille extérieure (2) dans une direction axiale (X-X) de l'instrument médical,
- un dispositif de coupe (4), lequel est agencé au niveau du dispositif de poussée, et
- un guide coulisse (5), lequel est agencé entre le dispositif de poussée et le dispositif de coupe (4), afin de convertir le mouvement axial du dispositif de poussée en un mouvement de pivotement du dispositif de coupe (4),
- dans lequel le dispositif de coupe (4) comprend une curette (40) avec un corps de base (41),
**caractérisé en ce que** la curette comprend en outre un œillet de coupe (42) annulaire avec une ouverture de passage (46).

2. Instrument selon la revendication 1, **caractérisé en ce que** la curette (40) est une curette coudée, dans lequel un premier axe longitudinal (L1) de l'œillet de coupe (42) est agencé en formant un angle par rapport à un deuxième axe longitudinal (L2) du corps de base (41).

3. Instrument selon la revendication 2, dans lequel un angle obtus (a) est prévu entre le premier axe longitudinal (L1) et le deuxième axe longitudinal (L2), en particulier dans une plage angulaire de 165° à 175°.

4. Instrument selon l'une quelconque des revendications précédentes, **caractérisé en ce que** le dispositif de poussée présente une tige de poussée (3) avec une tête de tige de poussée (32), au niveau de laquelle l'axe de pivotement (6) est agencé.

5. Instrument selon l'une quelconque des revendications précédentes, **caractérisé en ce que** le guide coulisse (5) comprend une rainure (50) agencée dans le corps de base (41) du dispositif de coupe (4) et un boulon (51) agencé au niveau de la douille extérieure (2), lequel est guidé dans la rainure.

6. Instrument selon la revendication 5, dans lequel la rainure (50) est une rainure arquée à rayon constant.

7. Instrument selon la revendication 6, dans lequel un coude de la curette (40) est dirigé dans la même direction, que celle dans laquelle s'étend la rainure (50) arquée.

8. Instrument selon l'une quelconque des revendications précédentes, dans lequel une longueur de l'œillet de coupe (42) en direction du premier axe longitudinal (L1) est à peu près aussi grande qu'une longueur du corps de base (41) en direction du deuxième axe longitudinal (L2).

9. Instrument selon l'une quelconque des revendications précédentes, dans lequel une liaison entre le dispositif de poussée et la curette (40) comprend une liaison tige-alésage.

10. Instrument selon l'une quelconque des revendications précédentes, dans lequel des arêtes de coupe (44, 45) sont prévues des deux côtés de l'ouverture de passage (46) de la curette (40).
